(19) Europäisches Patentamt
European Patent Office
Office européen des brevets

(11) Publication number: **0 146 350 A2**

# (12) EUROPEAN PATENT APPLICATION

(21) Application number: 84308692.7

(22) Date of filing: 13.12.84

(51) Int. Cl.⁴: **A 61 K 7/13**

(30) Priority: 15.12.83 GB 8333431

(43) Date of publication of application: 26.06.85 Bulletin 85/26

(84) Designated Contracting States: AT BE CH DE FR GB IT LI NL SE

(71) Applicant: UNILEVER PLC, Unilever House Blackfriars P O Box 68, London EC4P 4BQ (GB)

(84) Designated Contracting States: GB

(71) Applicant: UNILEVER NV, Burgemeester s'Jacobplein 1 P.O. Box 760, NL-3000 DK Rotterdam (NL)

(84) Designated Contracting States: BE CH DE FR IT LI NL SE AT

(72) Inventor: Nillson, Agne, Rydbergs vag 23, Staffanstorp (SE)

(74) Representative: Tonge, Robert James et al, UNILEVER PLC Patents Division P.O. Box 68 Unilever House, London EC4P 4BQ (GB)

(54) **Hair treatment composition.**

(57) A product for colouring and simultaneously conditioning the hair comprises two separately packaged components, an oxidiser and a base, which are intended to be mixed before application to the hair. The oxidiser component includes an oxidising agent and a mono-long-chain quaternary ammonium conditioning agent, and the base component includes an oxidation hair dye intermediate and ammonia to adjust the pH of the base component to a value of from 10 to 12.

EP 0 146 350 A2

HAIR TREATMENT COMPOSITION

The invention relates to a product for use in the colouring and simultaneous conditioning of hair.

Existing practice in the oxidative dyeing of hair includes the application to hair of an oxidation dye intermediate such as a p-diamine, together with an oxidising agent such as hydrogen peroxide. Usually, the dye intermediate and the oxidising agent are packaged separately and then mixed together immediately prior to application to the hair.

It has long been appreciated that although such a treatment is capable of imparting to the hair a permanent or semi-permanent colour of the desired hue, the condition of the hair is generally poor, it being difficult to comb out after the dyeing treatment. This problem can be eased by application of a conditioner which is usually applied after dyeing to render the hair more manageable.

The simultaneous dyeing and conditioning of hair has been proposed by Bristol-Myers Company who in British Patent 1 569 845 advocated the use of a di-long chain quaternary ammonium compound which is stored together with an oxidation dye intermediate and an alkaline agent such as ammonia, or an organic amine such as monoethanolamine or aminomethyl propanol, until required for application to the hair, at which point an oxidising agent such as hydrogen peroxide is added in order to effect oxidative dyeing of the hair.

In testing this proposal, it has been shown that although an improvement in the condition of the dyed hair is apparent provided that the prepacked mixture of the oxidative dye intermediate, the di-long chain quaternary ammonium compound and ammonia or organic amine is reasonably freshly prepared, prolonged storage of this mixture can lead to deterioration of the effectiveness as a conditioning agent of the di-long chain quaternary ammonium compound, such that the benefits of conditioning become less noticeable when an "aged" mixture is used.

It has also been shown that the Bristol-Myers composition can produce gradual but uncontrolled over-darkening of the hair following repeated application, particularly when organic amines are used as alkalizing agents, in view of the difficulty in rinsing such amines from the hair after dyeing has been effected.

It has been discovered that the aforementioned disadvantages of instability and over-darkening can be avoided by employing a hair dye composition in the form of two separate components, the first comprising an oxidising agent together with a conditioning agent, and the second comprising an oxidation dye intermediate together with ammonia in an amount sufficient to provide an alkaline pH.

Surprisingly, the application to the hair of a mixture of these components not only imparts to the hair a good conditioning effect, but also provides for controlled dyeing of the hair, such that repeated application at regular intervals to light or blonde hair achieves a greater degree of control in achieving a desirable shade, without over-darkening, than can be experienced when repeatedly using conventional oxidative dyeing techniques.

Accordingly, the invention provides a product for use in the colouring of hair which comprises an oxidiser component and a separate base component, which components are intended to be mixed together before application to the hair, the oxidiser component comprising in admixture an oxidising agent and a quaternary ammonium compound having the structure:

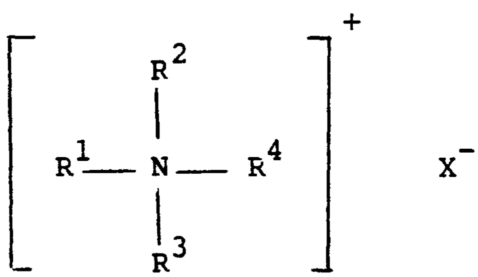

in which $R^1$ is a long chain aliphatic hydrocarbon radical having from 10 to 26 carbon atoms; and

$R^2$, $R^3$ & $R^4$ are each short chain aliphatic hydrocarbon radicals having from 1 to 5 carbon atoms; and

X is an anion.

the base component comprising in admixture an oxidation hair dye intermediate and ammonia as an alkalising agent

in an amount sufficient to adjust the base component to a pH of from 10 to 12.

The oxidiser component of the product accordingly includes an oxidising agent which preferably comprises hydrogen peroxide in an amount of from 2 to 10% by weight of the total product; this is equivalent from 5 to 25% by weight of 35% hydrogen peroxide as is available commercially. Alternatively, the oxidiser can comprise as the oxidising agent urea peroxide, sodium perborate, sodium percarbonate or sodium persulphate in an amount of from 0.5 to 20% by weight of the total weight of the product.

The oxidiser component of the product also includes, as a hair conditioning agent, a quaternary ammonium compound which will normally be one or more compounds having the structures:

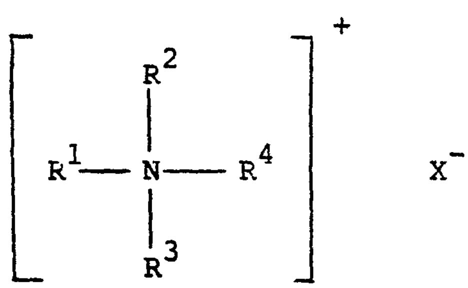

in which $R^1$ is a long chain aliphatic hydrocarbon radical having from 10 to 26 carbon atoms;

$R^2$, $R^3$ & $R^4$ are each short chain aliphatic hydrocarbon radicals having from 1 to 5 carbon atoms; and

X is an anion.

Examples of $R^1$ may, for example, be n-decyl, lauryl, myristyl, palmityl, stearyl, behenyl, cerotyl, $\Delta^9$-decylenyl, $\Delta^9$-dodecylenyl, palmitoleyl, oleyl, linoleyl, linolenyl, erucyl and lanolinyl.

Examples of $R^2$, $R^3$ and $R^4$ may, for example, be methyl, ethyl, n-propyl, i-propyl, n-butyl, sec. butyl, tert. butyl and n-pentyl.

Examples of the anion X include inorganic and organic anions, such as hydroxide, chloride, bromide, iodide, fluoride, sulphate, phosphate, sulphonate, acetate, n-propionate, lactate, gluconate, a lower alkyl sulphate, such as $SO_4R^5$, where $R^5$,is lower alkyl having from 1 to 5 carbons, for example $-SO_4CH_3$; $-SO_4C_2H_5$; $-SO_4(CH_2)_2CH_3$; $-SO_4CH(CH_3)_2$.

Examples of preferred quaternary ammonium compounds include stearyl trimethyl ammonium chloride, lauryl trimethyl ammonium chloride, hydrogenated beef tallow trimethyl ammonium chloride, cetyl trimethyl ammonium chloride, cetyl triethyl ammonium chloride, cetyl triethyl ammonium ethyl sulphate, Quaternium -33 which has the structure:

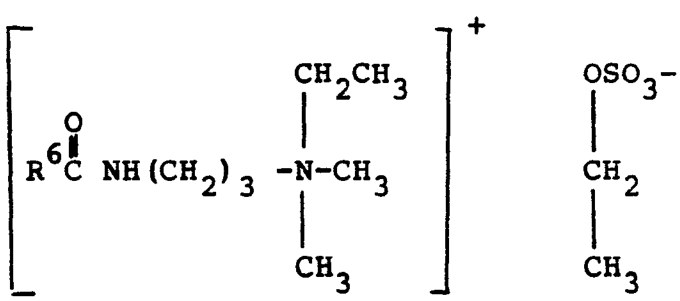

where $R^6$ represents the lanolic acid residue, having the empirical formula $C_{11}H_{22}O$-, which includes a mixture of similar acids derived from the hydrolysis of lanolin.

The preferred quaternary ammonium compounds are cetyl trimethyl ammonium chloride and Quaternium -33.

The amount of the quaternary ammonium compound to be employed in the product will generally be from 0.05% to 5% by weight of the total product, that is the weight of the product formed by mixing the oxidiser component with the base component.

The base component of the product accordingly includes an oxidation hair dye intermediate which can for example be any of those that are disclosed in Tables IV, V and VI on pages 308-310 of "Cosmetics Science and Technology", 2nd Edition, Volume 2, edited by Edward Sagarin, Interscience Publishers Inc., New York, 1972, or in Tables VIII, IX and X on pages 497-499 of "The Chemistry of Synthetic Dyes" edited by K Venkataraman, Volume V, Academic Press, New York, & London 1971. These publications are herein incorporated by reference.

Particular examples of oxidation hair dye intermediates are those having the structure:

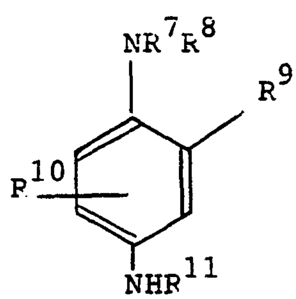

or their acid addition salts, in which

$R^7$ is alkyl or hydroxyalkyl
$R^8$ is hydrogen or hydroxyalkyl
$R^9$ is hydrogen, alkyl, alkoxy or halogen, and

$R^{10}$ is hydrogen, alkyl, alkoxy or halogen;
$R^{11}$ is hydrogen, alkyl or hydroxyalkyl;
provided that $R^8$ is hydrogen when $R^9$ is alkyl, alkoxy or halogen and provided also that at least two of $R^7$, $R^8$, $R^9$ or $R^{10}$ are other than hydrogen.

When $R^7$, $R^9$, $R^{10}$ or $R^{11}$, is alkyl, typical examples include methyl, ethyl, n-propyl, isopropyl, n-butyl, sec. butyl, tert. butyl, n-amyl, isoamyl and n-hexyl.

When $R^7$, $R^8$ or $R^{11}$ is hydroxyalkyl, typical examples include mono- and polyhydroxyalkyl radicals such as 2-hydroxyethyl, 2-hydroxypropyl; 3-hydroxypropyl; tri(hydroxymethyl)methyl; 1,3-dihydroxy-2-methylpropyl; 2,3-dihydroxypropyl; and 1,3-dihydroxy-2-propyl.

When $R^9$ or $R^{10}$ is halogen, it can be chloride, bromide, iodide or fluoride.

When $R^9$ or $R^{10}$ is alkoxy, the alkoxy group will usually contain from 1 to 6 carbon atoms, for example, methoxy, ethoxy, n-propoxy, isopropoxy, n-butoxy, n-amyloxy, isoamyloxy and isobutoxy.

Specific examples of oxidation hair dye intermediates having the above structures are:

p-phenylenediamine;
2-methyl-p-phenylenediamine;
2,6-dimethyl-p-phenylenediamine;
2-ethyl-p-phenylenediamine;
2-n-propyl-p-phenylenediamine;
2-isopropyl-p-phenylenediamine;
2-n-butyl-p-phenylenediamine;
N-ethyl-p-phenylenediamine;

N,N'-bis(ethyl)-p-phenylenediamine;
N-(n-propyl)-N'-(ethyl)-p-phenylenediamine;
N-methyl-2-amino-5-methyl-p-phenylenediamine;
N-methyl-2-methyl-5-amino-p-phenylenediamine;
N,N'-bis(ethyl)-2-ethyl-p-phenylenediamine;
N-(2-hydroxyethyl)-p-phenylenediamine;
N-(2-hydroxyethyl)-2-methyl-p-phenylenediamine;
N-(2-hydroxyethyl)-3-methyl-p-phenylenediamine;
N-(2-hydroxymethyl)-N'-(2-hydroxyethyl)-p-phenylenediamine;
N-(2-hydroxymethyl)-N'-(2-hydroxyethyl)-2-methyl-p-phenylenediamine;
N-(2-hydroxymethyl)-N'-(2-hydroxyethyl)-2-methyl-p-phenylenediamine;
N-ethyl-N-hydroxyethyl-p-phenylenediamine and its hydrochloride;
N,N'-bis(2-hydroxyethyl)-p-phenylenediamine and its hydrochloride or sulfate;
N,N'-bis(2-hydroxyethyl)-3-methyl-p-phenylenediamine and its hydrochloride or sulfate;
N,3-dimethyl-p-phenylenediamine and its hydrochloride;
N-hydroxyethyl-2-methyl-p-phenylenediamine and its hydrochloride;
N-dimethyl-p-phenylenediamine;
N-methyl-3-methoxy-p-phenylenediamine;
N-methyl-2-methoxy-p-phenylenediamine;
N-methyl-3-chloro-p-phenylenediamine;
N,N'-bis (2,3-dihydroxypropyl)-p-phenylene diamine.

Further particular examples of oxidation hair dye intermediates include:

p-aminophenol;
p-aminodiphenylamine;
4,4'-diaminodiphenylamine; and 2,5-diaminopyridine.

The amount of the oxidation hair dye intermediate to be employed in the product will generally be from 0.1 to 6%, preferably from 0.5 to 3% by weight of the total product.

It is further contemplated that these oxidation dye intermediates may also be used together with other oxidation dye intermediates which couple chemically with them under oxidative conditions. These coupling components comprise a well-known class of compounds in the hair dye art which are known to react oxidatively (i.e. with the aid of an oxidising agent) with p-phenylenediamine compounds to produce dyes. Examples of couplers are given in Tables III, IV and V and pages 483 & 484 of Venkataraman (Supra.)

The base component of the product also includes ammonium hydroxide as an alkalising agent in an amount sufficient to adjust the pH value of the base component to a value which will normally be from 8 to 12.

The oxidiser and the base components of the product can each also optionally contain other ingredients such as water, organic solvents, thickening agents, antioxidants, surfactants and perfume.

The oxidiser component and the base component can each also include water in an amount sufficient to ensure that each of the essential ingredients of the product are in solution, so facilitating intimate mixing of the components immediately prior to applying the product to the hair. The amount of water present in each component can accordingly form the balance of ingredients present in each component and will normally form from 50 to 99.35%, preferably from 60 to 95% by weight of the total product.

When the product of the invention is required for dyeing hair, the oxidiser and base components of the product are usually thoroughly mixed together and applied to the hair and allowed to remain in contact with the hair at an ambient temperature of about 20°C for a time sufficient for the oxidiser and base to react together and dye the hair. The normal time for this reaction is from 20 to 30 minutes, but an application (reaction) time of from 10 to 60 minutes can if desired be used. The hair can then be shampooed or simply rinsed with water to remove surplus dyestuff and other ingredients from the hair.

The following example illustrates the invention.

The oxidiser component of the product had the following formulation:

| | % w/w |
|---|---|
| cetostearyl alcohol | 2 |
| microcrystalline wax | 1 |
| mineral oil | 0.2 |
| Quaterium 33 | 0.5 |
| cetyltrimethyl ammonium chloride | 1 |
| perfume | 0.5 |
| 2-ethyl-1,3-hexane diol | 0.5 |
| hydrogen peroxide (35%) | 18 |
| water | to 100 |

The base component of the product had the following formulation:

| | % w/w |
|---|---|
| 2,4-diaminophenitol | 0.1 |
| resorcinol | 0.8 |
| p-phenylenediamine | 1 |
| sodium sulphite | 0.2 |
| EDTA | 0.1 |
| ethanol | 13 |
| linoleic diethanolamide | 5 |
| ammonium hydroxide (28% $NH_3$) | 4 |
| water | to 100 |

pH of base component of product was 12.

Equal volumes of the oxidiser and base component solutions of the product were mixed together thoroughly and immediately massaged into the hair (natural blonde) and allowed to remain in contact with the hair for a period of 20 minutes at 20°C.

The hair was rinsed with water and dried. It had acquired a rich golden brown colour and had a very soft texture indicative of superior conditioning.

Repeated application of the product to the hair at weekly intervals did not produce any further significant darkening of the rich golden brown colour.

Example 2

The oxidiser component of the product can have the following formulation:

| | % w/w |
|---|---|
| ceto-stearyl alcohol | 2 |
| microcrystalline wax | 1 |
| mineral oil | 0.2 |
| stearyl trimethyl ammonium chloride | 1.5 |
| perfume | 0.5 |
| 2-ethyl-1,3-hexane diol | 0.5 |
| hydrogen peroxide (35%) | 20 |
| water | to 100 |

The base component of the product can have the following formulation:

| | % w/w |
|---|---|
| 2,4-diamino phenitol | 0.1 |
| resorcinol | 0.8 |
| N-ethyl-N-hydroxyethyl-p-phenylene-diamine hydrochloride | 1.9 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

## Example 3

The oxidiser component of the product can have the following formulation:

| | % w/w |
|---|---|
| ceto-stearyl alcohol | 2 |
| microcrystalline wax | 1 |
| mineral oil | 0.2 |
| lauryl trimethyl ammonium chloride | 3 |
| perfume | 0.5 |
| 2-ethyl-1,3-hexane diol | 0.5 |
| hydrogen peroxide (35%) | 15 |
| water | to 100 |

The base component of the product can have the following formulation:

| | % w/w |
|---|---|
| 2,4-diamino phenitol | 0.1 |
| resorcinol | 0.8 |
| N,N-bis(2-hydroxyethyl)-p-phenylene-diamine sulphate | 0.9 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

## Example 4

The oxidiser component of the product can have the following formulation:

| | % w/w |
|---|---|
| ceto-stearyl alcohol | 2 |
| microcrystalline wax | 1 |
| mineral oil | 0.2 |
| hydrogenated beef tallow trimethyl-ammonium chloride | 2.4 |
| perfume | 0.5 |
| 2-ethyl-1,3-hexane diol | 0.5 |
| hydrogen peroxide (35%) | 17 |
| water | to 100 |

The base component of the product can have the following formulation:

| | % w/w |
|---|---|
| 2,4-diamino phenitol | 0.1 |
| resorcinol | 0.8 |
| N,N-bis(2-hydroxyethyl)-3-methyl-p-phenylenediamine hydrochloride | 2 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

## Example 5

The oxidiser component of the product can have the following formulation:

| | % w/w |
|---|---|
| ceto-stearyl alcohol | 2 |
| microcrystalline wax | 1 |
| mineral oil | 0.2 |
| cetyl triethyl ammonium chloride | 1.7 |
| perfume | 0.5 |
| 2-ethyl-1,3-hexane diol | 0.5 |
| hydrogen peroxide (35%) | 12 |
| water | to 100 |

The base component of the product can have the following formulation:

| | % w/w |
|---|---|
| 2,4-diamino phenitol | 0.1 |
| resorcinol | 0.8 |
| N,3-dimethyl-p-phenylenediamine hydrochloride | 0.8 |
| sodium sulphite | 0.2 |
| EDTA | 0.1 |
| ethanol | 13 |
| lincleic diethanolamine | 5 |
| ammonium hydroxide (28% $NH_3$) | qs |
| water | to 100 |

The pH of the base component of the product should be between pH 10 and 12.

Example 6

The oxidiser component of the product can have the following formulation:

| | % w/w |
|---|---|
| ceto-stearyl alcohol | 2 |
| microcrystalline wax | 1 |
| mineral oil | 0.2 |
| cetyl triethyl ammonium ethyl sulphate | 2.5 |
| perfume | 0.5 |
| 2-ethyl-1,3-hexane diol | 0.5 |
| sodium persulphate | 8 |
| water | to 100 |

The base component of the product can have the following formulation:

| | % w/w |
|---|---|
| 2,4-diamino phenitol | 0.1 |
| resorcinol | 0.8 |
| N-hydroxyethyl-2-methyl-p-phenylene-diamine hydrochloride | 1.2 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

## Example 7

The oxidiser component of the product can have the following formulation:

| | % w/w |
|---|---|
| ceto-stearyl alcohol | 2 |
| microcrystalline wax | 1 |
| mineral oil | 0.2 |
| cetyl triethyl ammonium chloride | 1.4 |
| Quaternium 33 | 0.6 |
| perfume | 0.5 |
| 2-ethyl-1,3-hexane diol | 0.5 |
| sodium percarbonate | 6 |
| water | to 100 |

The base component of the product can have the following formulation:

| | % w/w |
|---|---|
| 2,4-diamino phenitol | 0.1 |
| resorcinol | 0.8 |
| N-dimethyl-p-phenylenediamine hydrochloride | 1.5 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

## Example 8

The oxidiser component of the product can have the following formulation:

| | % w/w |
|---|---|
| ceto-stearyl alcohol | 2 |
| microcrystalline wax | 1 |
| mineral oil | 0.2 |
| stearyl trimethyl ammonium chloride | 1.2 |
| Quaternium 33 | 0.8 |
| perfume | 0.5 |
| 2-ethyl-1,3-hexane diol | 0.5 |
| sodium perborate | 5 |
| water | to 100 |

The base component of the product can have the following formulation:

| | % w/w |
|---|---|
| 2,4-diamino phenitol | 0.1 |
| resorcinol | 0.8 |
| N-methyl-3-methoxy-p-phenylenediamine sulphate | 0.9 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

Example 9

The oxidiser component of the product can have the following formulation:

| | % w/w |
|---|---|
| ceto-stearyl alcohol | 2 |
| microcrystalline wax | 1 |
| mineral oil | 0.2 |
| cetyl triethyl ammonium ethyl sulphate | 1.2 |
| Quaternium 33 | 1.6 |
| perfume | 0.5 |
| 2-ethyl-1,3-hexane diol | 0.5 |
| urea peroxide | 12 |
| water | to 100 |

The base component of the product can have the following formulation:

| | % w/w |
|---|---|
| 2,4-diamino phenitol | 0.1 |
| resorcinol | 0.8 |
| N-methyl-2-methoxy-p-phenylenediamine sulphate | 1.5 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

Example 10

The oxidiser component of the product can have the following formulation:

| | % w/w |
|---|---|
| ceto-stearyl alcohol | 2 |
| microcrystalline wax | 1 |
| mineral oil | 0.2 |
| lauryl trimethyl ammonium chloride | 1 |
| cetyl trimethyl ammonium chloride | 1 |
| perfume | 0.5 |
| 2-ethyl-1,3-hexane diol | 0.5 |
| hydrogen peroxide (35%) | 13 |
| water | to 100 |

The base component of the product can have the following formulation:

| | % w/w |
|---|---|
| 2,4-diamino phenitol | 0.1 |
| resorcinol | 0.8 |
| N-methyl-3-chloro-p-phenylenediamine hydrochloride | 1 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

## Example 11

The oxidiser component of the product can have the following formulation:

| | % w/w |
|---|---|
| ceto-stearyl alcohol | 2 |
| microcrystalline wax | 1 |
| mineral oil | 0.2 |
| hydrogentated beef tallow trimethyl ammonium chloride | 1.5 |
| Quaternium 33 | 0.5 |
| perfume | 0.5 |
| 2-ethyl-1,3-hexane diol | 0.5 |
| hydrogen peroxide (35%) | |
| water | to 100 |

The base component of the product can have the following formulation:

| | % w/w |
|---|---|
| 2,4-diamino phenitol | 0.1 |
| resorcinol | 0.8 |
| N,N-bis(2,3-dihydroxypropyl)-p-phenylenediamine sulphate | 1.3 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

Example 12

The oxidiser component of the product can have the following formulation:

| | % w/w |
|---|---|
| ceto-stearyl alcohol | 2 |
| microcrystalline wax | 1 |
| mineral oil | 0.2 |
| stearyl trimethyl ammonium chloride | 1.5 |
| perfume | 0.5 |
| 2-ethyl-1,3-hexane diol | 0.5 |
| hydrogen peroxide (35%) | 20 |
| water | to 100 |

The base component of the product can have the following formulation:

| | % w/w |
|---|---|
| 2,4-diamino phenitol | 0.1 |
| resorcinol | 0.8 |
| 2-methyl-p-phenylenediamine hydrochloride | 1.9 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

## Example 13

The oxidiser component of the product can have the following formulation:

| | % w/w |
|---|---|
| ceto-stearyl alcohol | 2 |
| microcrystalline wax | 1 |
| mineral oil | 0.2 |
| lauryl trimethyl ammonium chloride | 3 |
| perfume | 0.5 |
| 2-ethyl-1,3-hexane diol | 0.5 |
| hydrogen peroxide (35%) | 15 |
| water | to 100 |

The base component of the product can have the following formulation:

| | % w/w |
|---|---|
| 2,4-diamino phenitol | 0.1 |
| resorcinol | 0.8 |
| 2,6-dimethyl-p-phenylenediamine sulphate | 0.9 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

Example 14

The oxidiser component of the product can have the following formulation:

| | % w/w |
|---|---|
| ceto-stearyl alcohol | 2 |
| microcrystalline wax | 1 |
| mineral oil | 0.2 |
| hydrogenated beef tallow trimethyl-ammonium chloride | 2.4 |
| perfume | 0.5 |
| 2-ethyl-1,3-hexane diol | 0.5 |
| hydrogen peroxide (35%) | 17 |
| water | to 100 |

The base component of the product can have the following formulation:

| | % w/w |
|---|---|
| 2,4-diamino phenitol | 0.1 |
| resorcinol | 0.8 |
| 2-ethyl-p-phenylenediamine hydrochloride | 2 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

## Example 15

The oxidiser component of the product can have the following formulation:

| | % w/w |
|---|---|
| ceto-stearyl alcohol | 2 |
| microcrystalline wax | 1 |
| mineral oil | 0.2 |
| cetyl triethyl ammonium chloride | 1.7 |
| perfume | 0.5 |
| 2-ethyl-1,3-hexane diol | 0.5 |
| hydrogen peroxide (35%) | 12 |
| water | to 100 |

The base component of the product can have the following formulation:

| | % w/w |
|---|---|
| 2,4-diamino phenitol | 0.1 |
| resorcinol | 0.8 |
| N-ethyl-p-phenylenediamine sulphate | 0.8 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

## Example 16

The oxidiser component of the product can have the following formulation:

| | % w/w |
|---|---|
| ceto-stearyl alcohol | 2 |
| microcrystalline wax | 1 |
| mineral oil | 0.2 |
| cetyl triethyl ammonium ethyl sulphate | 2.5 |
| perfume | 0.5 |
| 2-ethyl-1,3-hexane diol | 0.5 |
| sodium persulphate | 8 |
| water | to 100 |

The base component of the product can have the following formulation:

| | % w/w |
|---|---|
| 2,4-diamino phenitol | 0.1 |
| resorcinol | 0.8 |
| N-(2-hydroxyethyl)-2-methyl-p-phenylene-diamine hydrochloride | 1.2 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

Example 17

The oxidiser component of the product can have the following formulation:

| | % w/w |
|---|---|
| ceto-stearyl alcohol | 2 |
| microcrystalline wax | 1 |
| mineral oil | 0.2 |
| cetyl triethyl ammonium chloride | 1.4 |
| Quaternium 33 | 0.6 |
| perfume | 0.5 |
| 2-ethyl-1,3-hexane diol | 0.5 |
| sodium percarbonate | 6 |
| water | to 100 |

The base component of the product can have the following formulation:

| | % w/w |
|---|---|
| 2,4-diamino phenitol | 0.1 |
| resorcinol | 0.8 |
| N-(2-hydroxymethyl)-N'-(2-hydroxyethyl)-2-methyl-p-phenylenediamine hydrochloride | 1.5 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

Example 18

The oxidiser component of the product can have the following formulation:

| | % w/w |
|---|---|
| ceto-stearyl alcohol | 2 |
| microcrystalline wax | 1 |
| mineral oil | 0.2 |
| stearyl trimethyl ammonium chloride | 1.2 |
| Quaternium 33 | 0.8 |
| perfume | 0.5 |
| 2-ethyl-1,3-hexane diol | 0.5 |
| sodium perborate | 5 |
| water | to 100 |

The base component of the product can have the following formulation:

| | % w/w |
|---|---|
| 2,4-diamino phenitol | 0.1 |
| resorcinol | 0.8 |
| p-aminophenol | 0.9 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

## Example 19

The oxidiser component of the product can have the following formulation:

| | % w/w |
|---|---|
| ceto-stearyl alcohol | 2 |
| microcrystalline wax | 1 |
| mineral oil | 0.2 |
| cetyl triethyl ammonium ethyl sulphate | 1.2 |
| Quaternium 33 | 1.6 |
| perfume | 0.5 |
| 2-ethyl-1,3-hexane diol | 0.5 |
| urea peroxide | 12 |
| water | to 100 |

The base component of the product can have the following formulation:

| | % w/w |
|---|---|
| 2,4-diamino phenitol | 0.1 |
| resorcinol | 0.8 |
| p-aminodiphenylamine | 1.5 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

Example 20

The oxidiser component of the product can have the following formulation:

| | % w/w |
|---|---|
| ceto-stearyl alcohol | 2 |
| microcrystalline wax | 1 |
| mineral oil | 0.2 |
| lauryl trimethyl ammonium chloride | 1 |
| cetyl trimethyl ammonium chloride | 1 |
| perfume | 0.5 |
| 2-ethyl-1,3-hexane diol | 0.5 |
| hydrogen peroxide (35%) | 13 |
| water | to 100 |

The base component of the product can have the following formulation:

| | % w/w |
|---|---|
| 2,4-diamino phenitol | 0.1 |
| resorcinol | 0.8 |
| 4,4'-diaminodiphenylamine | 1 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

Example 21

The oxidiser component of the product can have the following formulation:

| | % w/w |
|---|---|
| ceto-stearyl alcohol | 2 |
| microcrystalline wax | 1 |
| mineral oil | 0.2 |
| hydrogentated beef tallow trimethyl ammonium chloride | 1.5 |
| Quaternium 33 | 0.5 |
| perfume | 0.5 |
| 2-ethyl-1,3-hexane diol | 0.5 |
| hydrogen peroxide (35%) | |
| water | to 100 |

The base component of the product can have the following formulation:

| | % w/w |
|---|---|
| 2,4-diamino phenitol | 0.1 |
| resorcinol | 0.8 |
| 2,5-diaminopyridine | 1.3 |
| sodium sulphite | 0.2 |
| EDTA | 0.1 |
| ethanol | 13 |
| linoleic diethanolamine | 5 |
| ammonium hydroxide (28% $NH_3$) | qs |
| water | to 100 |

The pH of the base component of the product should be between pH 10 and 12.

CLAIMS:

1. An aqueous product for use in the colouring of hair which comprises an oxidiser component and a separate basic component, which components are intended to be mixed together before application to the hair, characterised in that

the oxidiser component comprises in admixture an oxidising agent and a quaternary ammonium compound having the structure:

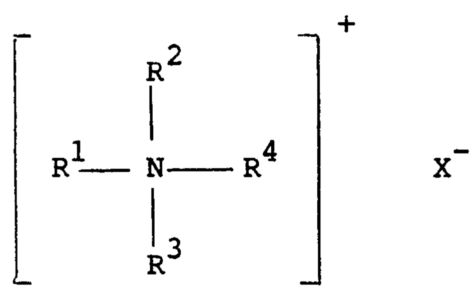

in which $R^1$ is a long chain aliphatic hydrocarbon radical having from 10 to 26 carbon atoms;

$R^2$, $R^3$ & $R^4$ are each short chain aliphatic hydrocarbon radicals having from 1 to 5 carbon atoms;

X is an anion; and
mixtures thereof; and

the base component comprises in admixture an oxidation hair dye intermediate and ammonia as an alkalising agent in an amount sufficient to maintain the base component at a pH of from 10 to 12.

2. A product according to claim 1, characterised in that the oxidising agent is hydrogen peroxide which forms from 2 to 10% by weight of the total product.

3. A product according to claim 1, characterised in that the oxidising agent is chosen from urea peroxide, sodium perborate, sodium percarbonate, sodium persulphate and mixtures thereof, and forms from 0.5 to 20% by weight of the total product.

4. A product according to claim 1, characterised in that the quaternary ammonium compound is chosen from

cetyl trimethyl ammonium chloride,
Quaternium 33 and mixtures thereof.

5. A product according to any preceding claim, characterised in that the quaternary ammonium compound forms from 0.05 to 5% by weight of the total product.

6. A product according to any preceding claim, characterised in that the oxidation hair dye intermediate is chosen from:

p-phenylenediamine;
2-methyl-p-phenylenediamine;
2,6-dimethyl-p-phenylenediamine;
2-ethyl-p-phenylenediamine;
2-n-propyl-p-phenylenediamine;
2-isopropyl-p-phenylenediamine;
2-n-butyl-p-phenylenediamine;
N-ethyl-p-phenylenediamine;
N,N'-bis(ethyl)-p-phenylenediamine;
N-(n-propyl)-N'-(ethyl)-p-phenylenediamine;
N-methyl-2-amino-5-methyl-p-phenylenediamine;
N-methyl-2-methyl-5-amino-p-phenylenediamine;
N,N'-bis(ethyl)-2-ethyl-p-phenylenediamine;
N-(2-hydroxyethyl)-p-phenylenediamine;

N-(2-hydroxyethyl)-2-methyl-p-phenylenediamine;
N-(2-hydroxyethyl)-3-methyl-p-phenylenediamine;
N-(2-hydroxymethyl)-N'-(2-hydroxyethyl)-p-phenylenediamine;
N-(2-hydroxymethyl)-N'-(2-hydroxyethyl)-2-methyl-p-phenylenediamine;
N-(2-hydroxymethyl)-N'-(2-hydroxyethyl)-2-methyl-p-phenylenediamine;
N-ethyl-N-hydroxyethyl-p-phenylenediamine and its hydrochloride;
N,N'-bis(2-hydroxyethyl)-p-phenylenediamine and its hydrochloride or sulfate;
N,N'-bis(2-hydroxyethyl)-3-methyl-p-phenylenediamine and its hydrochloride or sulfate;
N,3-dimethyl-p-phenylenediamine and its hydrochloride;
N-hydroxyethyl-2-methyl-p-phenylenediamine and its hydrochloride;
N-dimethyl-p-phenylenediamine;
N-methyl-3-methoxy-p-phenylenediamine;
N-methyl-2-methoxy-p-phenylenediamine;
N-methyl-3-chloro-p-phenylenediamine;
N,N-bis (2,3-dihydroxypropyl)-p-phenylene diamine;
and mixtures thereof.

7. A product according to any of claims 1 to 5, characterised in that the oxidation hair dye intermediate is chosen from:

p-aminophenol;
p-aminodiphenylamine;
4,4'-diaminodiphenylamine; 2,5-diaminopyridine;
and mixtures thereof.

8. A product according to any preceding claim, characterised in that the oxidation hair dye intermediate forms from 0.1 to 6% by weight of the total product.